# FASCICULE DE BREVET EUROPEEN

(11) **EP 1 963 859 B1**
(45) Date de publication et mention de la délivrance du brevet: **01.12.2010**
(21) Numéro de dépôt: 06841854.0
(22) Date de dépôt: 05.12.2006
(51) Int. Cl.: G01N 33/569, G01N 33/50, G01N 33/58, G01N 15/14

(54) **METHODE DE DISCRIMINATION D'AU MOINS DEUX POPULATIONS CELLULAIRES ET APPLICATION**
VERFAHREN ZUR UNTERSCHEIDUNG VON WENIGSTENS ZWEI ZELLPOPULATIONEN UND ANWENDUNG
METHOD OF DISCRIMINATING AT LEAST TWO CELL POPULATIONS, AND APPLICATION

(30) Priorité: 20.12.2005 FR 0512948
(43) Date de publication de la demande: 03.09.2008
(73) Titulaire: HORIBA ABX SAS, F-34000 Montpellier (FR)
(72) Inventeur: LACOMBE, Francis, F-33600 PESSAC (FR); BELLOC, Francis, F-33600 Pessac (FR); VERIAC, Sylvie, F-34070 Montpellier (FR); LEFEVRE, Didier, F-34980 Saint Clement de Rivière (FR)
(74) Mandataire: Galup, Cédric Olivier Nicolas
(86) Numéro de dépôt international: PCT/FR2006/002645
(87) Numéro de publication internationale: WO 2007/080245

(56) Documents cités:
- EP-A- 0 633 462
- WO-A-03/016866
- HAWLEY T S ET AL: "Four-color flow cytometric detection of retrovirally expressed red, yellow, green, and cyan fluorescent proteins." BIOTECHNIQUES. MAY 2001, vol. 30, no. 5, mai 2001 (2001-05), pages 1028-1034, XP002963379 ISSN: 0736-6205
- MAURER D ET AL: "A single laser flow cytometry method to evaluate the binding of three antibodies" JOURNAL OF IMMUNOLOGICAL METHODS 1990 NETHERLANDS, vol. 135, no. 1-2, 1990, pages 43-47, XP009066500 ISSN: 0022-1759
- KOVIT PATTANAPANYASAT ET AL: "FLOW CYTOMETRIC TWO-COLOR STAINING TECHNIQUE FOR SIMULTANEOUS DETERMINATION OF HUMAN ERYTHROCYTE MEMBRANE ANTIGEN AND INTRACELLULAR MALARIAL DNA" CYTOMETRY, ALAN LISS, NEW YORK, US, vol. 13, no. 2, janvier 1992 (1992-01), pages 182-187, XP000371957 ISSN: 0196-4763
- BEAVIS ANDREW J ET AL: "Detection of cell-surface antigens using antibody-conjugated fluorospheres (ACF): Application for six-color immunofluorescence" BIOTECHNIQUES, vol. 21, no. 3, 1996, pages 498-503, XP001247049 ISSN: 0736-6205
- BROWN MICHAEL ET AL: "Flow cytometry: Principles and clinical applications in hematology" CLINICAL CHEMISTRY, vol. 46, no. 8B Part 2, 22 mai 2000 (2000-05-22), pages 1221-1229, XP002380657 ISSN: 0009-9147

## Description

La présente invention concerne une méthode de détection, de discrimination et de comptage d'éléments biologiques présents dans un liquide, utilisant les principes de la cytométrie en flux, adaptable aux appareils utilisés en routine en hématologie.

Les analyseurs automatiques en hématologie (automates d'hématologie), présents sur le marché apportent de plus en plus de possibilités d'analyses et de classifications des éléments analysés.

La mesure de fluorescence, déjà largement répandue en cytométrie en flux, est principalement utilisée pour la classification des éléments par immunophénotypage. Dans les automates d'hématologie utilisés en routine, elle est surtout dédiée à la mise en évidence de colorants supra vitaux utilisés comme sondes moléculaires pour la quantification des acides nucléiques ou autres composants cellulaires.

L'utilisation de sondes immunologiques en hématologie de routine n'est pas encore généralisée bien que quelques essais embryonnaires aient déjà vu le jour chez différents constructeurs.

Par exemple, la société BAYER (Bayer Diagnostics, Tarrytown, New York, USA), au travers du BAYER-TECHNICON H*1 a été la première à proposer, dans le typage lymphocytaire, l'utilisation de cocktails d'anticorps pour la détermination des différents types de lymphocytes. Dans ce cas, la mesure des expressions antigéniques se faisait non pas par fluorescence mais par la mesure d'absorbance lumineuse générée par un composé d'avidine-peroxydase présentant une grande affinité pour la biotine, elle-même couplée sur un anticorps. Ledit anticorps est spécifique des antigènes cibles des molécules de surface spécifiques des types cellulaires caractérisés (CD4, CD8, CD2, CD 19).

L'ABBOTT CD4000, de la société ABBOTT (Abbott Laboratories, Abbott Park, Illinois, USA), propose une analyse en utilisant deux longueurs d'onde de fluorescence pour la réalisation, entre autres, d'immunophénotypages. Le brevet WO 98/02727 de la société Abbott Laboratories décrit un appareillage de ce type, permettant de réaliser le marquage avec un anticorps sur un échantillon de sang total. ABBOTT décrit de façon détaillée dans le brevet un appareillage destiné à la réalisation de réactions anticorps - antigène sur un échantillon de sang total.

Une méthode de phénotypage simple, rapide, efficace et spécifique doit pouvoir être utilisée dans des automates très simples, de type automates d'hématologie, permettant de travailler avec un nombre limité de détecteurs. Même si les technologies sont sensiblement les mêmes, les automates de routine utilisés en hématologie présentent des caractéristiques différentes des cytomètres en flux, notamment en terme de nombre de paramètres de mesure qui sont réduits au strict nécessaire, particulièrement dans les mesures de routine. De plus, le coût étant un facteur limitant très important, la simplification des appareils et des analyses qu'ils permettent d'effectuer ne peuvent qu'aller dans le sens de l'économie globale.

La cytométrie en flux est une technologie permettant de mesurer simultanément de multiples paramètres correspondants à différentes caractéristiques physiques d'un élément biologique comme par exemple une cellule ou un organite cellulaire. Les éléments biologiques sont entraînés dans un flux liquide et l'appareil enregistre le comportement de chacun d'eux quand ils passent dans la cuve de mesure devant une source lumineuse.

Cette technologie est en fait la combinaison de trois systèmes :
1) Système fluidique : Flux laminaire qui permet aux éléments biologiques en suspension de passer un à un devant la lumière dans la cuve de mesure.
2) Système optique : Rayon laser ou autre source lumineuse et différents filtres qui permettent de sélectionner les longueurs d'ondes appropriées tant en excitation qu'en émission.
3) Système électronique : PMT (photomultiplicateur) ou photodiode, qui capte la lumière émise, permettant sa transformation en signal électrique puis en signal numérique.

En résumé, la source lumineuse permet de générer une lumière qui va passer à travers des lentilles et éclairer les éléments biologiques défilant dans une cuve de mesure. Au contact de l'élément biologique, une partie de la lumière est diffusée. Cette lumière passe au travers de plusieurs lentilles et autres diaphragmes et est focalisée sur un capteur de type photodiode pour générer la mesure de FSC (Forward Scatter). Cette mesure, dans la gamme d'angles choisie, donne une indication sur la taille de l'élément biologique.

Une autre partie de la lumière est déviée de façon orthogonale et passe au travers d'un autre ensemble de lentilles, et d'un jeu de miroirs semi-réfléchissants, pour être mesurée au niveau d'un capteur pour générer le signal SSC (Side Scatter). Cette mesure de lumière orthogonale, donne une indication sur la densité de l'élément biologique ainsi que sur sa granularité (structure).

Enfin, des mesures de l'intensité lumineuse peuvent être réalisées grâce à autant de tubes photomultiplicateurs ou de photodiodes que de longueurs d'onde et de signaux optiques à analyser.

Le passage des éléments biologiques dans un cytomètre nécessite au préalable une étape au cours de laquelle l'élément biologique est rendu détectable, et donc marqué, par une sonde, spécifique d'une structure ou d'une fonction dudit élément biologique, ladite sonde ayant elle-même préalablement été rendue détectable.

Lorsque le marqueur de la sonde est une molécule fluorescente celle-ci peut absorber un photon dans une gamme de longueur d'onde qui lui est spécifique (spectre d'excitation). Ainsi excitée, la molécule fluorescente, va retourner à son état fondamental, et restituer un photon avec une énergie plus faible. Une molécule fluorescente possède donc un spectre de longueurs d'onde d'excitation qui lui est propre et dans lequel elle va absorber de l'énergie pour la ré émettre sous forme de fluorescence (fluorescence émise), selon un spectre d'émission également caractéristique. La longueur d'onde de la fluorescence émise est toujours plus grande (fréquence plus faible) que la longueur d'onde d'excitation.

Les signaux de diffusion (FSC, SSC) et de fluorescence vont être transformés en signaux électriques par les détecteurs adaptés, puis analysés par un système informatique.

Ainsi en cytométrie en flux, il est communément associé une bande de longueurs d'onde de fluorescence à chaque sonde ou cocktail de sondes utilisé(s). Chaque sonde est donc couplée avec un fluorochrome (marqueur fluorescent), dont les signaux sont mesurés sur un seul canal de fluorescence, que ledit fluorochrome soit greffé sur un ou plusieurs types de sondes. Par exemple, le document EP0633462 décrit une méthode de discrimination de différentes cellules lymphocytaires par le marquage simultané desdites populations par trois anticorps monoclonaux rendus détectables par trois fluorochromes différents. Les documents Hawley et al. (BioTechniques 30, (2001) N°5, 1028-1034) et Maurer et al (Journal of immunological Methods, (1990) N°135, 43-47) décrivent également chacun l'utilisation de la cytométrie de flux pour détecter plusieurs marqueurs.

La diminution des coûts pourrait passer par la mise en routine de paramètres d'immunophénotypage avec un nombre restreint de canaux de mesure de fluorescence en plus des paramètres physiques classiques choisis parmi la diffusion dans l'axe (Forward Scatter ou FSC), la diffusion orthogonale (Side Scatter ou SSC), le volume par impédancemétrie, etc.

La possibilité de sommer les réponses de fluorescence permettrait d'exprimer plusieurs marquages différents sur un même canal de mesure et ainsi de multiplier les capacités d'analyse d'un système et donc son rapport prix/performance, sans pour autant augmenter la complexité de l'appareil par l'ajout de canaux de mesure.

La difficulté lorsque plusieurs sondes sont conjuguées au même fluorochrome, est que l'expression des caractéristiques biologiques reconnues respectivement par les différentes sondes, va directement influencer la quantité de fluorescence totale émise.

Ainsi, par exemple, si une caractéristique biologique est fortement présente (par exemple un antigène à la surface d'une cellule) la quantité de sondes marquées et fixées, sera proportionnelle à la quantité de ladite caractéristique biologique. En conséquence la quantité de fluorescence émise par ladite sonde, elle-même proportionnelle à la quantité de caractéristiques biologiques ayant fixées ladite sonde marquée, sera élevée.

Parallèlement, si une autre caractéristique biologique du même échantillon est faiblement représentée, la quantité de fluorescence émise par la sonde correspondante, conjuguée au même fluorochrome, sera trop faible et pourra passer inaperçue car masquée par la fluorescence de la première sonde.

Bien entendu, les mêmes difficultés sont rencontrées lors de l'utilisation d'autres types de marquages.

L'un des buts de la présente invention est précisément de permettre la détection nette et sans ambiguïté d'au moins trois sondes par l'utilisation de seulement deux moyens de détection. Cela implique donc qu'au moins deux sondes soient détectées par un seul et même moyen de détection. Ceci peut être réalisé si l'on utilise trois sondes différentes, chacune reconnaissant et se fixant à l'un des éléments biologiques à détecter ou à l'une des caractéristiques de (ou des) l'élément(s) biologique(s), chacune des sondes étant elle-même rendue détectable par un marqueur différent, deux desdits marqueurs présentant deux spectres d'émission ayant au moins une partie commune (spectres d'émission couvrants) et le troisième présentant un spectre d'émission n'ayant essentiellement aucune partie commune avec les deux autres (spectre non couvrant). Les deux (ou n) marqueurs ayant un spectre d'émission couvrant sont mesurés par un premier moyen de détection, le marqueur ayant le spectre d'émission non couvrant étant lui mesuré par un second moyen de détection.

Ainsi, la présente invention consiste :
(1) - à équilibrer les différences d'expression antigénique ou de caractéristique cellulaire par le rendement d'émission de n sondes couplées à n marqueurs mesurés en une seule et même bande de détection ;
(2) - à créer un rapport entre les signaux issus du second moyen de détection d'une part, et les signaux issus du premier moyen de détection d'autre part, afin de l'utiliser comme axe constitutif d'une matrice ;
(3) - à combiner en deux dimensions le rapport issu de l'item (2) d'une part, et les signaux issus du premier moyen de détection d'autre part, dans le but d'améliorer la représentation graphique, et donc d'améliorer la caractérisation et la quantification des éléments biologiques d'intérêt.

Par "élément biologique" on entend selon l'invention par exemple une cellule eucaryote ou procaryote, un groupe de cellules, un fragment cellulaire.

Par "caractéristique biologique", on entend un composant spécifique de l'élément biologique étudié, par exemple, un composant biologique de la cellule tel qu'un organite cellulaire, une protéine, un lipide, un glucide, ou encore un acide nucléique (ARN ou ADN).

Par "sonde", selon l'invention on entend tout moyen permettant d'identifier spécifiquement une caractéristique biologique ou chimique présente dans ou sur les éléments biologiques étudiés. Lesdits moyens utilisables selon l'invention sont parfaitement connus en particulier dans les domaines de la biologie cellulaire, de la biologie moléculaire, de l'immunologie et de la cytométrie en flux. On peut citer sans être limitatif, les anticorps, les acides nucléiques (ADN ou d'ARN), les composés chimiques, particulièrement les composés chimiques intercalants, ceux réactifs à l'environnement ionique (H⁺, Ca⁺⁺, etc.), les lectines, ligands ou autres colorants de viabilité cellulaire.

Par "marqueur de la sonde" on entend tout composé qui de par sa nature est directement détectable soit visuellement, soit à l'aide d'un appareil adéquat, soit détectable après excitation. Un tel composé une fois couplé à la sonde rend celle-ci détectable. On peut citer les molécules fluorescentes qu'il s'agisse par exemple de composés chimiques ou de molécules biologiques comme des protéines. Tous ces produits sont disponibles sur le marché et proposés par la plupart des distributeurs de produits chimiques de laboratoire, comme par exemple les sociétés Sigma, Aldrich, Fluka, Riedel de Haen, etc...

Il est notable que dans une même expérience de discrimination de populations d'éléments biologiques utilisant la méthode selon l'invention, il soit possible d'utiliser des sondes de natures différentes (anticorps conjugués et acides nucléiques marqués ou viabilité cellulaire par exemple) dès lors que leur détection puisse être réalisée selon les critères définis selon l'invention (au moins deux spectres d'émission couvrants et un spectre non couvrant).

Par "moyen de détection", on entend selon l'invention tout moyen de détecter, voire de quantifier, le marqueur de la sonde. Ledit moyen de détection est bien entendu caractéristique de la méthode utilisée pour rendre la sonde détectable. Ledit moyen de détection est dans tous les cas, composé de l'ensemble des composants physiques capables de détecter le marqueur d'une sonde particulière. Par exemple, s'il s'agit d'un marqueur émettant de la fluorescence, le moyen de détection sera l'ensemble composé des lentilles optiques d'observation de l'échantillon, des filtres spatiaux (diaphragme, "pin hole", etc), des filtres spectroscopiques (dichroïques, interférentiels) spécialement arrangés pour ne laisser passer vers le capteur que la partie du spectre dite "bande de détection" spécifique du ou des marqueurs à mesurer et du capteur opto-électronique lui même (photodiode, tube photomultiplicateur...). Ce capteur sera ensuite connecté électroniquement à la chaîne d'acquisition électronique qui réalisera le traitement du signal en analogique ou/et numérique et enfin le traitement informatique des données via un ordinateur.

Il peut aussi s'agir par exemple d'un détecteur de fluorescence si la sonde est marquée à l'aide d'un marqueur fluorescent ou si la sonde est elle même fluorescente, ou encore d'un spectrophotomètre si la sonde est un marqueur absorbant dans une partie spécifique du spectre chromatique de la lumière d'excitation.

Par "spectre d'émission" on entend selon l'invention la répartition de l'intensité de fluorescence en fonction de la longueur d'onde dans une bande caractéristique du marqueur ou de la sonde elle même. Généralement un tel spectre présente un pic d'intensité correspondant au maximum d'émission. La détection pourra se faire dans une plage comprise entre deux longueurs d'ondes, encadrant généralement une longueur d'onde intermédiaire donnée correspondant au pic de fluorescence maximale émise (pic de détection maximale).

Par "bande de détection" on entend une gamme de longueurs d'onde choisie dans le spectre d'émission du ou des marqueurs considérés (Par exemple, s'il s'agit d'un marqueur émettant de la fluorescence, la bande de détection sera une bande choisie de préférence autour du pic maximal d'émission de fluorescence). Cette bande de détection est définie par le filtrage spectral incorporé dans le moyen de détection, par l'intermédiaire d'au moins un filtre interférentiel ou passe bande éventuellement assemblé derrière un ou plusieurs miroirs dichroïques.

Par "spectres d'émission couvrants", on entend que, dans un même moyen de détection, pour deux marqueurs différents, leurs spectres d'émission présentent une zone commune. Par exemple s'il s'agit de deux marqueurs émettant de la fluorescence, leurs spectres d'émission seront dit spectres d'émission couvrants lorsqu'ils présenteront une plage d'émission commune comprise entre deux longueurs d'onde communes à leurs deux spectres d'émission. Généralement de tels spectres présentent des pics d'émission maximale distincts.

Par "spectre d'émission essentiellement non couvrant", on entend que pour trois marqueurs différents dont deux présentent des spectres d'émission couvrants, le troisième présente un spectre d'émission n'ayant aucune zone de recouvrement significative avec aucun des spectres des deux autres marqueurs.

A la lecture de ce qui précède on comprend donc que si l'on utilise trois sondes qui reconnaissent trois caractéristiques biologiques différentes, marquées de trois marqueurs ayant chacun un spectre d'émission différents, mais dont deux sont couvrants et un non couvrant, la méthode n'utilise que deux moyens de détection dans la mesure où les marqueurs à spectres couvrants ont une bande de détection commune.

C'est cela qui fait la grande originalité de la méthode objet de l'invention par rapport aux méthodes connues dans l'art antérieur qui pour détecter trois caractéristiques biologiques utilisent trois sondes présentant trois spectres d'émission indépendants qui sont analysés par trois moyens de détection différents.

Pour les marqueurs couvrants, le choix judicieux des marqueurs dans leur aspect détection, conduira à choisir deux marqueurs pouvant répondre, l'un plus efficacement que l'autre, dans la même bande de détection.

Selon une variante de l'invention, le rendement d'émission dans la bande de détection pourra être choisi pour être inversement proportionnel à l'expression des caractéristiques biologiques considérées. Le choix de la bande de détection sera aussi déterminé en fonction de cette expression pour ne pas masquer une expression positive faible (c'est la première phase d'équilibrage de l'invention).

Ainsi, par exemple, pour un antigène peu exprimé on choisira un marqueur présentant un rendement d'émission élevé dans la bande de détection choisie et pour un antigène très exprimé on choisira un marqueur présentant un rendement d'émission peu élevé dans la même bande de détection. Cela permet de contrôler les quantités de marqueurs mesurés afin d'équilibrer les émissions en proportion inverse du niveau d'expression des antigènes ou sondes considérés.

La méthode selon l'invention permet de discriminer et compter des éléments biologiques de façon simple, rapide, efficace. Elle permet en outre d'utiliser des appareils simples, ce qui a pour conséquence une diminution des coûts tant de fabrication que d'utilisation et de maintenance. De plus l'analyse automatisée est facilitée.

Ainsi l'invention a pour objet une méthode de discrimination d'au moins deux populations d'éléments biologiques porteuses de caractéristiques spécifiques, éventuellement présentes dans un échantillon, comprenant
- le marquage simultané desdites populations d'éléments biologiques par trois sondes différentes, détectables ou rendues détectables par trois marqueurs différents, dont deux desdits marqueurs (marqueurs couvrants ou MC) présentent chacun un spectre d'émission, lesdits spectres d'émission se recouvrant et le troisième (marqueur non couvrant ou MnC) présente un spectre d'émission ne recouvrant essentiellement pas les spectres des deux autres marqueurs (spectre d'émission non couvrant) ;
- la mesure par tout moyen approprié, de la quantité totale de marqueur non couvrant (qMnC), en une bande de détection choisie dans le spectre d'émission dudit marqueur non couvrant ;
- la mesure par tout moyen approprié, de la quantité totale de marqueur couvrant (qMC) en une bande de détection commune aux spectres d'émission desdits marqueurs couvrants,
- l'établissement pour chaque élément biologique analysé, du rapport (R) de la - quantité totale de marqueur non couvrant par rapport à la quantité totale de marqueur couvrant [R = (qMnC) / (qMC)]
- l'établissement par tout moyen d'un diagramme présentant le rapport (R) en fonction de la quantité d'éléments biologiques marqués par les marqueurs couvrants [R = f(qMC)].

Cette méthode est également caractérisée en ce qu'elle comprend, en outre, une dernière étape de quantification par tout moyen approprié, généralement un ordinateur, des éléments biologiques identifiés sur le ou les dits diagrammes et des données statistiques correspondantes.

La méthode selon l'invention est une méthode qui permet la discrimination et le comptage d'éléments biologiques répondant positivement ou non à un critère donné. La méthode n'a pas pour but de quantifier le nombre de sondes fixées par élément biologique. La méthode permet de mettre en évidence sans ambiguïté, dans un échantillon, particulièrement un échantillon biologique, des populations d'éléments biologiques présentant la (ou les) caractéristique(s) recherchée(s).

Conformément à la dernière étape de la méthode selon l'invention, l'analyse d'une matrice bi-paramétrique d'ordonnée R et d'abscisse qMC permet d'améliorer la représentation graphique, et donc d'améliorer la caractérisation et la quantification des éléments biologiques d'intérêt.

Selon l'invention, l'échantillon peut être un échantillon biologique naturel, particulièrement un échantillon biologique naturel liquide. Il peut également être une suspension cellulaire non naturelle, comme par exemple un milieu de culture. On peut citer sans être limitatif, comme liquide biologique naturel le sang, l'urine, un tissu dissocié, la moelle osseuse, le liquide céphalo-rachidien, du liquide pleural ou du liquide synovial, le produit résultant d'un procédé d'aphérèse ou comme suspension cellulaire synthétique, un milieu de culture de cellules ou de microorganismes.

Selon l'invention les éléments biologiques contenus dans l'échantillon peuvent être des cellules, eucaryotes ou procaryotes, ou un mélange des deux, ou des fragments desdites cellules, des organites. Les sondes utilisables selon la méthode de l'invention peuvent être, identiques ou différentes, des anticorps, des acides nucléiques (ADN ou ARN), ou encore toute sonde moléculaire comme par exemple des colorants reconnaissant spécifiquement des acides nucléiques, les substrats enzymatiques ou encore des marqueurs spécifiques de protéines, des ligands de récepteurs, des molécules sensibles à l'environnement ionique (sondes à pH, Ca⁺⁺, etc.) ou tout autre molécule spécifique de la caractéristique biologique ciblée.

Lorsque les sondes sont des anticorps, ceux-ci peuvent être des anticorps monoclonaux ou polyclonaux, naturels ou recombinants, humains ou animaux.

Les sondes, si elles ne sont pas détectables naturellement, devront, pour être détectables, être conjuguées à un marqueur pouvant être reconnu par le moyen de détection, dans la bande de détection choisie.

Selon l'invention, les marqueurs servant à rendre détectable la sonde, peuvent être des composés chimiques détectables aptes à être greffés sur les sondes, des marqueurs intercalants ou non des acides nucléiques, ou encore des marqueurs spécifiques de protéines ou de tout autre molécule spécifique de la caractéristique biologique ciblée. A cet égard on peut citer des colorants fluorescents ou encore absorbants tel que : l'Alexa Fluor^{®} 350, l'Alexa Fluor^{®} 488, l'Alexa Fluor^{®} 532, l'Alexa Fluor^{®} 633, l'Alexa Fluor^{®} 647, l'Alexa Fluor^{®} 660, l'Alexa Fluor^{®} 680, l'allophycocyanine, l'aminométhylcoumarine acetic acid, le Cy2^{®}, le Cy 5.1^{®}, le Cy 5^{®}; le Cy 5.5^{®}, le dichlorofluorescéine (DCFH), le dihydrorhodamine (DHR), "l'enhanced GFP" (EGFP), le Fluo-3, le FluorX^{®}, la fluorescéine, la 5-maleimide fluorescéine, l'isothiocyanate de fluorescéine (FITC), le PerCP, la r-Phycoérythrine (PE), le tandem r-Phycoérythrine-Cyanine 5 ou SpectralRed^{®} ou CyChrome^{®}, la r-Phycoérythrine-Cyanine 5.5 (PE-CY 5.5^{®}), la r-Phycoérythrine-Cyanine 7 (PE-CY 7^{®}), la r-Phycoérythrine-Texas Red-x^{®}, le Red 613^{®}, la Rhodamine 110, la Rhodamine 123, le S65L, le S65T, l'Isothiocyanate de tétraméthylerhodamine, le Texas-Red-x^{®}, le TruRed^{®}, l'indo 1, les nano cristaux (Quantum Dots), le Fura 2, le Fura 3, le quin, le DS Red ainsi que les marqueurs spécifiques notamment des acides nucléiques (qu'ils soient sous forme d'ADN ou d'ARN), comme par exemple des intercalants ou autres colorants de viabilité cellulaire tels que le bromure d'éthidium ou encore le thiazole orange, le thiazole blue et leurs dérivés, la thioflavine S, la thioflavine T, la thioflavine TCN^{®}, le diéthyl-quinolythocyanine iodide (DEQTC), le TOTO-1^{®}, le TO-PRO-1^{®}, ou encore le YOYO-1^{®}, l'Hoechst^{®} 33258, l'Hoechst^{®} 33342, l'Hoechst^{®} 34580, le diamidino phénylindole (DAPI), l'iodure de propidium, la pyronine Y, la 7-Aminoactinomycine D (7AAD), l'acridine orange, l'auramine O, la calceine, le New Methylene Blue, l'olamin-O, l'Oxazine 750, le bleu astra, , le SYTOX^{®} Green, le SYTO 11^{®}, le SYTO 12^{®}, le SYTO 13^{®}, le SYTO 16^{®}, le SYTO 18^{®}, le SYTO 80^{®}, le SYTO 81^{®}....

Dans une forme particulière de réalisation de l'invention, lorsque des caractéristiques cellulaires, par exemple des antigènes, sont reconnues par des sondes rendues détectables par des marqueurs couvrants, on peut choisir une première caractéristique cellulaire dite générique, c'est-à-dire présente et caractéristique de tous les éléments biologiques considérés. L'expression de cette caractéristique cellulaire étant normalement élevée dans les éléments biologiques, le marqueur qui sera greffé à la sonde le reconnaissant pourra être celui qui répondra plus faiblement dans la bande de détection commune aux deux marqueurs couvrants (MC). Une telle sonde permet de vérifier que l'on s'intéresse bien aux bonnes populations cellulaires.

La seconde caractéristique cellulaire reconnue par une sonde rendue détectable par l'autre marqueur couvrant, pourra être une caractéristique cellulaire moins exprimée sur une population seulement des éléments biologiques étudiées. Le marqueur qui sera greffé à ladite sonde devra être un marqueur qui répondra plus fortement dans la bande de détection commune aux deux marqueurs couvrants (MC).

A la troisième caractéristique cellulaire étudiée, correspond une sonde marquée par un troisième marqueur qui lui est non couvrant (MnC) et qui sera détecté dans une deuxième bande de détection, différente de la première.

La méthode selon l'invention peut être mise en oeuvre dans tout appareil permettant la détection des marqueurs. On citera à cet égard à titre d'exemple la détection de fluorescence qu'elle soit émise par un colorant conjugué à une sonde moléculaire telle qu'un anticorps ou directement par un colorant supravital fluorescent tel qu'un colorant intercalant ou non, spécifique des acides nucléiques, ou encore la détection d'absorbance ou de diffusion lumineuse notamment à une gamme de longueurs d'onde définie du spectre, que ces mesures soient faites par extinction ou diffusion de la gamme de longueurs d'onde ou par spectrométrie d'une bande choisie de l'ultraviolet à l'infrarouge. Cette absorbance pouvant être celle générée soit par un colorant conjugué à une sonde moléculaire telle qu'un anticorps ou directement par un colorant supravital tel qu'un colorant intercalant ou non, spécifique des acides nucléiques.

L'invention a aussi pour objet l'utilisation de la méthode décrite précédemment pour discriminer au moins deux populations cellulaires porteuses de caractéristiques spécifiques et éventuellement présentes dans un échantillon. Une application de la méthode peut être envisagée dans le diagnostic médical, comme par exemple dans le domaine des leucémies lymphoïdes chroniques (LLC) et des leucémies aigues (LA) à l'aide d'anticorps anti-CD45, anti-CD19 et anti-CD5 ; dans le domaine de la détection de la maladie résiduelle (Minimum Residual Desease, MRD) et de la différenciation leucocytaire à l'aide d'anticorps anti-CD45, anti-CD16 et anti-CD11b ; dans le domaine des progéniteurs hématopoïétiques à l'aide d'anticorps anti-CD45, anti-CD34, anti-CD33 et 7-AAD ou DAPI ou autre marqueur de viabilité ; dans le domaine de l'inflammation et de l'activation cellulaire à l'aide d'anticorps anti-CD45, anti-CD64 et anti-CD163 ; dans le domaine de la détection ou du suivi d'infection au virus HIV à l'aide d'anticorps anti-CD45, anti-CD8 ou anti-CD4 et anti-CD3 ; dans le domaine de la Leucémie Aiguë Lymphoblastique de type b (LALb) de l'enfant ; dans le domaine de l'observation de la moelle osseuse à l'aide d'anticorps anti-CD45, anti-CD19 et anti-CD10 ou encore le domaine de la différenciation des précurseurs des lymphocytes B (hématogones) à l'aide d'anticorps anti-CD19, anti-CD10 et anti-CD38. Ces descriptions n'étant ni exhaustives ni limitatives puisque dans l'état actuel des connaissances médicales.

D'autres caractéristiques de l'invention apparaîtront à la lecture des figures et des exemples qui suivent et qui ne sont donnés qu'à titre illustratif sans limitation de l'invention.

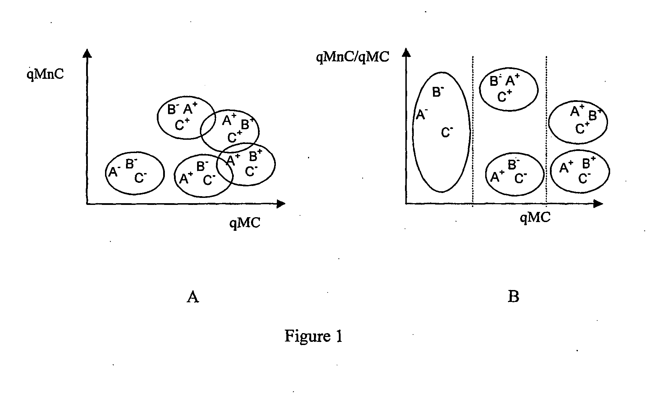

Ainsi la Figure 1 est une représentation schématique de phénotypes d'éléments biologiques dans laquelle A et B sont des marqueurs couvrants et C est un marqueur non couvrant, qui correspondent respectivement à des sondes (SA, SB et SC), rendues détectables qui reconnaissent des caractéristiques particulières (CPA, CPB et CPC) des éléments biologiques considérés.

La partie 1A montre une représentation graphique classique en cytométrie en flux de la fluorescence de A+B (qMC) en fonction de la fluorescence de C (qMnC).

La partie 1B montre une représentation graphique obtenue selon l'invention en cytométrie en flux du rapport de la fluorescence de C (qMnC) rapportée à la fluorescence totale de A+B (qMC) [qMnC/qMC] en fonction de la fluorescence totale de A+B (qMC) [qMnC/qMC = f(qMC)].

La mise en oeuvre de l'invention permet une distinction sur un même axe de fluorescence (l'axe des abscisses) entre les éléments biologiques exprimant (A+ B+) d'une première part, (A- B-) d'une deuxième part, et (A+ B-) ou (A- B+) d'une troisième part:

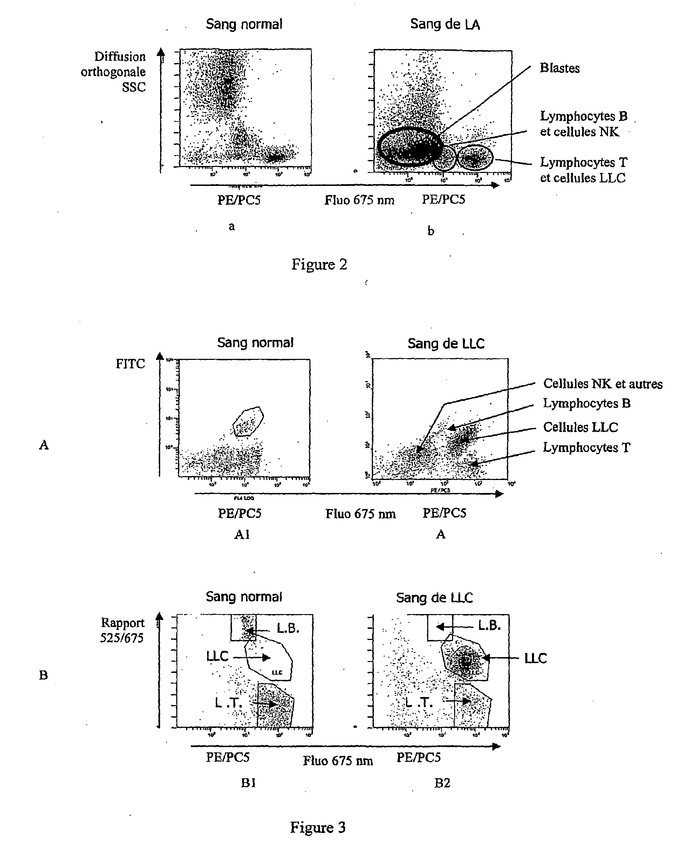

La Figure 2 présente la méthode selon l'invention appliquée à l'analyse des leucocytes sanguins dans un échantillon de sang normal (2a) et dans un échantillon de sang de patient atteint de leucémie aiguë (2b) après marquage avec un anticorps anti-CD45 couplé à la r-phycoérythrine (PE), un anticorps anti-CD5 couplé à la phycoérythrine-cyanine 5 (PC5) et un anticorps anti-CD19 couplé à la FITC. L'expression du CD45 seul permet d'identifier les blastes présents dans cette pathologie.

La figure 3 représente des échantillons de sang normal et de sang de malade atteint de leucémie lymphoïde chronique (A2 et B2). Les Figures 3A1 et 3A2 présentent les résultats de l'analyse par cytométrie en flux, les Figures 3B1 et 3B2 présentent les mêmes résultats après application de la méthode selon l'invention.

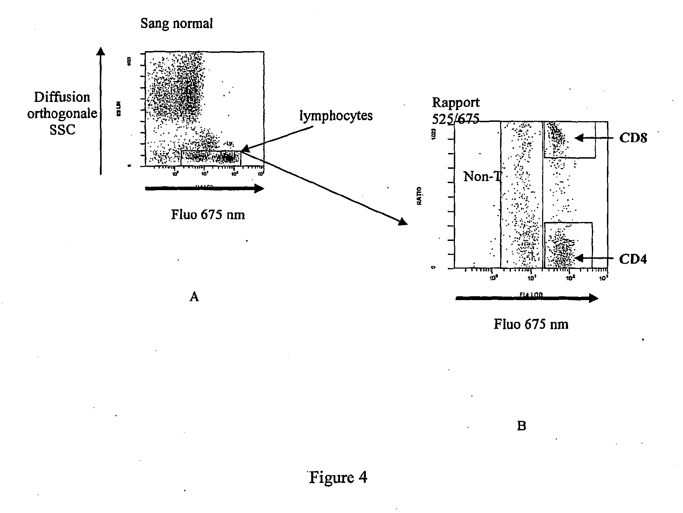

La Figure 4 présente la méthode selon l'invention appliquée à l'analyse des lymphocytes T sanguins après marquage avec un anticorps anti-CD45 couplé à la PE, un anticorps anti-CD3 couplé au PC5 et un anticorps anti-CD8 couplé à la FITC. La Figure 3A présente les résultats de l'analyse par cytométrie en flux, la Figure 3B présente les mêmes résultats après application de la méthode selon l'invention.

### Exemple 1 :

Les pathologies les plus fréquentes en onco-hématologie sont les différents types de leucémies aiguës (LA) et les hémopathies lymphoïdes B dont la leucémie lymphoïde chronique (LLC) présente une incidence en constante progression (30/10⁶).

La leucémie aiguë (LA) peut être caractérisée par un envahissement médullaire par prolifération de cellules hématopoïétiques malignes.

La leucémie lymphoïde chronique (LLC) peut, elle, être caractérisée par une prolifération de cellules lymphocytaires monoclonales, cellules lymphoïdes de la lignée B atypiques.

Les blastes leucémiques des LA sont caractérisés par une expression modérée du CD45 comparée à celle des lymphocytes et monocytes normaux du sang.

Les lymphocytes anormaux de la LLC de type B sont caractérisés par la co-expression aberrante des antigènes CD5 et CD19 sur la même cellule alors que les lymphocytes normaux expriment soit le CD5 (lymphocytes de la lignée T) soit le CD19 (lymphocytes de la lignée B).

Les phénotypes de ces cellules peuvent être résumés sur le tableau ci-dessous :

| Antigènes | CD19 | CD45 | CD5 |
|---|---|---|---|
| Lymphocytes T normaux | - | ++ | ++ |
| Lymphocytes B normaux | + | ++ | - |
| Blastes de LA | | +/- | |
| Lymphocytes B de LLC | + | ++ | + |

Jusqu'à présent, l'analyse et l'identification de ces différentes populations dans un échantillon sanguin par cytométrie en flux nécessitent l'emploi de trois anticorps différents dirigés contre les trois antigènes CD5, CD 19 et CD45 et de la diffusion orthogonale SSC. Pour cette analyse, chaque anticorps est couplé à un fluorochrome différent et chaque expression est mesurée à une longueur d'onde différente à l'aide de photomultiplicateurs différents.

L'application de la méthode selon l'invention permet de proposer un système de marquage et d'analyse par cytométrie permettant de détecter et de quantifier dans un même échantillon de sang et lors d'une même mesure, la présence éventuelle de cellules leucémiques à l'aide de deux photomultiplicateurs seulement et de la diffusion orthogonale SSC.

Sur un cytomètre en flux, le filtrage optique des signaux lumineux est optimisé pour un fluorochrome donné de façon à mesurer sa fluorescence dans une bande de longueurs d'onde centrée sur son maximum d'émission : la fluorescéine (FITC) est généralement analysée à 525 nm, la r-phycoérythrine (PE) à 575 nm et le tandem r-phycoérythrine-cyanine 5 (PC5) à 675 nm, ceci pour des largeurs de bande d'environ 30 nm.

Les spectres d'émission de PE et de PC5 sont couvrants. Ils ont par exemple en commun la longueur d'onde 675 nm à laquelle la PE a un rendement de fluorescence beaucoup plus faible que le PC5.L'une des originalités de la méthode selon l'invention réside dans la mesure de la fluorescence de l'un des fluorochromes (PE) à une longueur d'onde différente de celle de son maximum d'émission, ici la mesure de PE à 675 nm. Ainsi, à cette longueur d'onde, il devient possible, en jouant sur des efficacités différentes du système de mesure vis à vis de deux fluorochromes choisis à cet effet, de compenser des différences importantes dans l'expression des antigènes analysés.

Ce principe est appliqué à l'analyse des leucocytes sanguins après marquage avec un anticorps anti-CD45 couplé à la PE (λmax = 575nm), un anticorps anti-CD5 couplé au PC5 (λmax = 675nm) et un anticorps anti-CD19 couplé à la FITC (λmax = 525nm).

Ces marquages sont analysés dans une première bande de détection centrée à 675 nm qui est la longueur d'onde d'émission maximale de PC5. Le spectre de la PE recouvre celui de PC5. L'analyse à 675 nm déterminera donc la quantité totale de fluorescence émise par PC5 et PE (qMC).

D'autre part, on analyse la quantité de fluorescence émise par l'anticorps anti-CD 19 couplé à la FITC à 525 nm (qMnC).

Les polynucléaires et les monocytes sont exclus de l'analyse par fenêtrage sur leurs propriétés de diffusion lumineuse.

### Protocole :

1) Dans un échantillon de sang total périphérique, on prélève une aliquote de sang de quelques microlitres (5 à 50) que l'on mélange avec une aliquote de quelques microlitres (3 à 50) de la solution des trois anticorps conjugués ci-dessus décrits.
2) Le mélange est mis en incubation à température ambiante ou à température régulée, à l'abri de la lumière, pendant une période de quelques minutes (1 à 30).
3) Après la période d'incubation, le mélange est additionné d'un réactif de lyse des globules rouges, pour obtenir une solution finale de sang à une concentration spécifique voulue (1/40, 1/80, 1/100, etc..).
4) La solution de sang lysé ainsi obtenue est laissée à incuber pendant quelques secondes dépendamment du réactif de lyse et de la température d'incubation (typiquement 15 à 30 secondes).
5) Puis la solution est injectée dans un ensemble de mesure du type cytomètre en flux pour mesurer sur chaque cellule traversant un faisceau optique (le plus souvent un laser 488nm pour les colorants spécifiés), les paramètres de diffusion dans l'axe FSC, diffusion orthogonale SSC, fluorescence verte à 525 nm (FL1) et fluorescence rouge à 675 nm (FL2). Les étapes 3 à 5 peuvent être réalisées automatiquement sur un appareil semi automatique de cytométrie en flux ou un automate d'hématologie spécialement conçu et les étapes 1 à 5 peuvent être automatisées sur un appareil automatique de cytométrie en flux spécialement conçu.

L'analyse de la fluorescence à 675 nm (FL1) en fonction de la diffusion orthogonale SSC (voir Figure 2 A) permet de discriminer:
- une population très peu fluorescente correspondant aux blastes de LA exprimant peu le CD45,
- une population d'une fluorescence intermédiaire correspondant aux lymphocytes exprimant le CD45 mais n'exprimant pas le CD5 (lymphocytes B et cellules NK) et
- une population très fluorescente correspondant aux lymphocytes exprimant en plus le CD5 (lymphocytes T et cellules de LLC).

Dans cette dernière population, l'analyse de la fluorescence à 525 nm permet d'identifier les cellules de LLC exprimant aussi le CD19.

L'application de la méthode selon l'invention permet de discriminer les lymphocytes non T, les lymphocytes T et les cellules de LLC en prenant en compte, pour chaque cellule, le rapport de la fluorescence à 525 nm sur la fluorescence à 675 nm (qMnC/qMC). Ce rapport s'exprime selon la table de vérité ci-dessous :

| CD19 | CD45 | CD5 | Rapport CD19/CD45+CD5 (qMnC/qMC) |
|---|---|---|---|
| - | ++ | ++ | - |
| + | ++ | - | ++ |
| + | ++ | + | +/- |

L'histogramme de distribution obtenu selon l'invention [(qMnC) / (qMC) = f(qMC)] (voir Figure 2C) permet d'identifier, de séparer et de quantifier trois populations très distinctement séparées.
- Les lymphocytes T normaux (L.T.) ne fluorescent pas à 525 nm mais fluorescent beaucoup à 675 nm ils présentent donc un rapport très faible.
- Les lymphocytes B normaux (L.B.) fluorescent à 525 nm avec une fluorescence intermédiaire à 675 nm, ils ont donc un rapport élevé.
- Les cellules de LLC expriment à la fois le CD5, le CD45 et le CD 19, fluorescent à 525 nm et à 675 nm et leur rapport est donc intermédiaire (Figure 2).
- Les cellules NK qui n'expriment ni le CD5 ni le CD 19 sont exclues de l'analyse sur la base de leur fluorescence à 675nm (CD45+, CD5- et CD19-).

Ainsi à partir d'un échantillon de sang, après marquage par trois anticorps fluorescents et lyse des hématies il est possible, en une seule analyse, de discriminer et de compter les lymphocytes T, les lymphocytes B, et les cellules NK par la mesure à deux longueurs d'onde de fluorescences seulement.

L'application de la méthode selon l'invention permet de discriminer les lymphocytes T et les cellules LLC (figure 2B) qui apparaissaient sous la forme d'un seul nuage dans le diagramme obtenu selon une analyse conventionnelle (figure 2A). Il en est de même pour les lymphocytes B et les cellules NK.

De plus, la présence éventuelle de blastes de leucémie aiguë ou de cellules de leucémie lymphoïde chronique peut être détectée et ces cellules peuvent être dénombrées.

L'utilisation de la méthode selon l'invention peut s'appliquer au diagnostic et au suivi des pathologies lymphoïdes chroniques B, des leucémies aiguës, et au suivi de la maladie résiduelle au cours du traitement.

### Exemple 2 :

La méthode selon l'invention peut aussi être appliquée à l'analyse des lymphocytes T sanguins après marquage avec un anti-CD45 couplé à la PE, un anti-CD3 couplé au PC5 et un anti-CD8 couplé à la FITC. (Figure 3).
1) Dans un échantillon de sang total périphérique, on prélève une aliquote de sang de quelques microlitres (5 à 50) que l'on mélange avec une aliquote de quelques microlitres (3 à 50) de la solution des trois anticorps conjugués ci-dessus décrits.
2) Le mélange est mis en incubation à température ambiante ou à température régulée, à l'abri de la lumière, pendant une période de quelques minutes (1 à 30).
3) Après la période d'incubation, le mélange est additionné d'un réactif de lyse des globules rouges, pour obtenir une solution finale de sang à une concentration spécifique voulue (1/40, 1/80, 1/100, etc..).
4) La solution ainsi obtenue est laissée à incuber pendant quelques secondes dépendantes du réactif de lyse et de la température d'incubation (typiquement 15 à 30 secondes).
5) Puis la solution est injectée dans un ensemble de mesure du type cytomètre en flux pour mesurer sur chaque cellule passant au travers d'un faisceau optique (le plus souvent un laser 488nm pour les colorants spécifiés), les paramètres de diffusion dans l'axe FSC, diffusion latérale SSC, fluorescence verte à 525 nm (FL1) et fluorescence rouge à 675 nm (FL2).

Les étapes 3 à 5 peuvent être réalisées automatiquement sur un appareil semi automatique de cytométrie en flux ou un automate d'hématologie spécialement conçu et les étapes 1 à 5 peuvent être automatisées sur un appareil automatique de cytométrie en flux spécialement conçu.

Les marquages à la PE et au PC5 sont analysés à 675 nm (qMC). Le marquage à la FITC est analysé à 525 nm (qMnC).

Les polynucléaires et les monocytes sont exclus de l'analyse par leurs propriétés de diffusion orthogonale SSC.

Le diagramme obtenu selon les méthodes classiques (Figure 3A) de la fluorescence à 675 nm en fonction de la diffusion orthogonale SSC permet de mettre en évidence un nuage représentant les populations moyennement fluorescentes de lymphocytes exprimant le CD45 mais n'exprimant pas le CD3 (lymphocytes B et cellules NK) et une population très fluorescente correspondant aux lymphocytes exprimant en plus le CD3 (lymphocytes T).

L'application de la méthode selon l'invention permet de parfaitement discriminer les différentes populations (Figure 3 B).

En particulier cela permet d'identifier, dans la population très fluorescente correspondant aux lymphocytes exprimant en plus le CD3, les lymphocytes CD8+.

Les lymphocytes T CD4+ ne fluorescent pas à 525 nm mais fluorescent beaucoup à 675 nm et ne présentent qu'un rapport 525/675 très faible.

Les lymphocytes T CD8+ fluorescent à 525 nm et à 675 nm, ils ont donc un rapport plus élevé.

Les cellules NK et les cellules B qui n'expriment pas le CD3 sont identifiées sur la base de leur fluorescence modérée à 675nm (Non T).

## Revendications

1. Méthode de discrimination et de comptage d'au moins deux populations d'éléments biologiques porteuses de caractéristiques biologiques spécifiques, éventuellement présentes dans un échantillon, comprenant
- le marquage simultané desdites populations d'éléments biologiques par trois sondes différentes, détectables ou rendues détectables par trois marqueurs différents, dont deux desdits marqueurs (marqueurs couvrants ou MC) présentent chacun un spectre d'émission, lesdits spectres d'émission se recouvrant et le troisième (marqueur non couvrant ou MnC) présente un spectre d'émission ne recouvrant pas les spectres des deux autres marqueurs (spectre d'émission non couvrant) ;
- la mesure par tout moyen approprié, de la quantité totale de marqueur non couvrant (qMnC), en une bande de détection choisie dans le spectre d'émission dudit marqueur non couvrant ;
- la mesure par tout moyen approprié, de la quantité totale de marqueurs couvrants (qMC) en une bande de détection commune aux spectres d'émission desdits marqueurs couvrants,
- l'établissement du rapport (R) de la quantité totale de marqueurs non couvrants à la quantité totale de marqueurs couvrants [R = (qMnC) / (qMC)] ;
- l'établissement par tout moyen d'un diagramme présentant le rapport (R) en fonction de la quantité totale d'éléments biologiques marqués par le marqueur couvrant [R = f(qMC)].

2. Méthode selon la revendication 1, **caractérisée en ce qu'**elle comprend en outre, une dernière étape de quantification par tout moyen des populations des éléments biologiques présents sur ledit diagramme.

3. Méthode selon l'une des revendications 1 ou 2, **caractérisée en ce que** le liquide biologique naturel est du sang, de l'urine, un tissu dissocié, de la moelle osseuse, du liquide céphalo-rachidien, du liquide pleural, du liquide synovial, du produit résultant d'un procédé d'aphérèse.

4. Méthode selon l'une quelconque des revendications 1 à 3, **caractérisée en ce que** les éléments biologiques éventuellement présents dans le liquide sont des cellules eucaryotes ou des cellules procaryotes, ou un mélange des deux, ou des fragments desdites cellules.

5. Méthode selon l'une quelconque des revendications 1 à 4, **caractérisée en ce que** les sondes, identiques ou différentes, sont des anticorps ou des acides nucléiques (ADN ou ARN) ou des colorants spécifiques des acides nucléiques ou des substrats enzymatiques ou encore des marqueurs spécifiques de protéines, des ligands de récepteurs ou des marqueurs sensibles à l'environnement ionique ou tout autre molécule caractéristique de l'élément biologique ciblé.

6. Méthode selon la revendication 5, **caractérisée en ce que** les anticorps sont des anticorps monoclonaux ou polyclonaux, naturels ou recombinants, humains ou animaux.

7. Méthode selon l'une quelconque des revendications 1 à 6, **caractérisée en ce que** les marqueurs servant à rendre détectables la sonde, sont des marqueurs, intercalants ou non, des acides nucléiques, ou encore des marqueurs spécifiques de protéines ou de tout autre molécule caractéristique de l'élément biologique ciblé.

8. Méthode selon l'une quelconque des revendications 1 à 7, **caractérisée en ce que** les marqueurs sont des marqueurs fluorescents ou non.

9. Méthode selon l'une quelconque des revendications 7 ou 8, **caractérisée en ce que** le marqueur est choisi parmi l'Alexa Fluor^{®} 350, l'Alexa Fluor^{®} 488, l'Alexa Fluor^{®} 532, l'Alexa Fluor^{®} 633, l'Alexa Fluor^{®} 647, l'Alexa Fluor^{®} 660, l'Alexa Fluor^{®} 680, l'allophycocyanine, l'aminométhylcoumarine acetic acid, le Cy2^{®}, le Cy 5.1^{®}, le Cy 5^{®}, le Cy 5.5^{®}, le dichlorofluorescéine (DCFH), le dihydrorhodamine (DHR), "l'enhanced GFP" (EGFP), le Fluo-3, le FluorX^{®}, la fluorescéine, la 5-maleimide fluorescéine, l'isothiocyanate de fluorescéine (FITC), le PerCP, la r-Phycoérythrine (PE), le tandem r-Phycoérythrine-Cyanine 5 ou SpectralRed^{®} ou CyChrome^{®}, la r-Phycoérythrine-Cyanine 5.5 (PE-CY 5.5^{®}), la r-Phycoérythrine-Cyanine 7 (PE-CY 7^{®}), la r-Phycoérythrine-Texas Red-x^{®}, le Red 613^{®}, la Rhodamine 110, la Rhodamine 123, le S65L, le S65T, l'Isothiocyanate de tétraméthylerhodamine, le Texas-Red-x^{®}, le TruRed^{®}, l'indo 1, les nano cristaux (Quantum Dots), le Fura 2, le Fura 3, le quin, le DS Red, les composés intercalants tel le bromure d'éthidium ou encore le thiazole orange, le thiazole blue, la thioflavine S, la thioflavine T, la thioflavine TCN^{®}, le diéthyl-quinolythocyanine iodide (DEQTC), le TOTO-1^{®}, le TO-PRO-1^{®}, ou encore le YOYO-1^{®}, l'Hoechst^{®} 33258, l'Hoechst^{®} 33342, l'Hoechst^{®} 34580, le diamidino phénylindole (DAPI), l'iodure de propidium, la pyronine Y, la 7-Aminoactinomycine D (7AAD), l'acridine orange, l'auramine O, la calceine, le New Methylene Blue, l'olamin-O, l'Oxazine 750, le bleu astra, , le SYTOX^{®} Green, le SYTO 11^{®}, le SYTO 12^{®}, le SYTO 13^{®}, le SYTO 16^{®}, le SYTO 18^{®}, le SYTO 80^{®}, le SYTO 81^{®}.

10. Utilisation de la méthode selon l'une quelconque des revendications précédentes pour discriminer au moins deux populations d'éléments biologiques porteuses de caractéristiques spécifiques et éventuellement présentes dans un échantillon.

## Claims

1. Method for discriminating between and counting at least two populations of biological elements bearing specific biological characteristics, optionally present in a sample, comprising
- the simultaneous labelling of said biological elements by three different probes, which are detectable or rendered detectable by three different labels (overlapping labels or OLs) each having an emission spectrum, said emission spectra overlapping each other and the third (non-overlapping label or nOL) has an emission spectrum not overlapping the spectra of the other two labels (non-overlapping emission spectrum);
- the measurement, by any appropriate means, of the total quantity of non-overlapping label (nOL) in a detection band chosen from the emission spectrum of said non-overlapping label;
- the measurement, by any appropriate means, of the total quantity of overlapping labels (qOL) in a detection band common to the emission spectra of said overlapping labels;
- the establishment of the ratio (R) of the total quantity of non-overlapping labels to the total quantity of overlapping labels [R = (qnOL): (qOL)];
- the establishment by any means of a diagram showing the ratio (R) as a function of the total quantity of biological elements labelled by the overlapping label [R = f(qOL)].

2. Method according to claim 1, **characterized in that** it also comprises a second stage of quantification by any means of the populations of the biological elements present in said diagram.

3. Method according to either of claims 1 or 2, **characterized in that** the natural biological fluid is blood, urine, a dissociated tissue, bone marrow, cerebrospinal fluid, pleural fluid, synovial fluid, product resulting from an apheresis process.

4. Method according to any one of claims 1 to 3, **characterized in that** the biological elements optionally present in the fluid are eukaryotic cells or prokaryotic cells, or a mixture of the two, or fragments of said cells.

5. Method according to any one of claims 1 to 4, **characterized in that** the probes, identical or different, are antibodies or nucleic acids (DNA or RNA) or dyes specific to nucleic acids or enzyme substrates or also labels specific to proteins, receptor ligands or labels sensitive to the ionic environment or any other molecule characteristic of the targeted biological element.

6. Method according to claim 5, **characterized in that** the antibodies are human or animal, natural or recombinant, monoclonal or polyclonal antibodies.

7. Method according to any one of claims 1 to 6, **characterized in that** the labels serving to render the probe detectable are intercalating or non-intercalating labels of the nucleic acids, or also labels specific to proteins or any other molecule characteristic of the targeted biological element.

8. Method according to any one of claims 1 to 7, **characterized in that** the labels are fluorescent or non-fluorescent labels.

9. Method according to any one of claims 7 or 8, **characterized in that** the label is chosen from Alexa Fluor® 350, Alexa Fluor® 488, Alexa Fluor® 532, Alexa Fluor® 633, Alexa Fluor® 647, Alexa Fluor® 660, Alexa Fluor® 680, allophycocyanin, aminomethylcoumarin acetic acid, Cy2®, Cy 5.1®, Cy 5®, Cy 5.5®, dichlorofluorescein (DCFH), dihydrorhodamine (DHR), "enhanced GFP" (EGFP), Fluo-3, FluorX®, fluorescein, fluorescein-5-maleimide, fluorescein isothiocyanate (FITC), PerCP, r-Phycoerythrin (PE), the r-Phycoerythrin Cyanine 5 or SpectralRed® or CyChrome® tandem, r-Phycoerythrin-Cyanine 5.5 (PE-CY 5.5 ), r-Phycoerythrin-Cyanine 7 (PE-CY 7 ), r-Phycoerythrin-Texas Red-X , Red 613 , Rhodamine 110, Rhodamine 123, S65L, le S65T, tetramethylrhodamine isothiocyanate, Texas-Red-X®, TruRed®, Indo-1, nanocrystals (Quantum Dots), Fura 2, Fura 3, Quin, DS Red, intercalating compounds such as ethidium bromide or also thiazole orange, thiazole blue, thioflavine S, thioflavine T, thioflavine TCN®, diethyl-quinolythocyanine iodide (DEQTC), TOTO-1®, TO-PRO-1®, or also YOYO-1®, Hoechst® 33258, Hoechst® 33342, Hoechst® 34580, diamidino phenylindole (DAPI), propidium iodide, pyronine Y, 7-aminoactinomycin D (7AAD), acridine orange, auramine O, calcein, New Methylene Blue, olamin-O, oxazine 750, Astra blue, SYTOX® Green, SYTO 11®, SYTO 12®, SYTO 13®, SYTO 16®, SYTO 18®, SYTO 80®, SYTO 81®.

10. Use of the method according to any one of the previous claims in order to discriminate between at least two populations of biological elements bearing specific characteristics and optionally present in a sample.

## Patentansprüche

1. Verfahren zum Unterscheiden und Zählen von wenigstens zwei Populationen von biologischen Trägerelementen mit spezifischen biologischen Charakteristiken, die eventuell in einer Probe vorliegen, das Folgendes beinhaltet:
- gleichzeitiges Markieren der genannten Populationen von biologischen Elementen mit drei verschiedenen Sonden, die mit drei verschiedenen Markern erkennbar sind oder erkennbar gemacht wurden, wobei zwei der genannten Marker (deckende Marker oder DM) jeweils ein Emissionsspektrum aufweisen, wobei sich die genannten Emissionsspektren überlappen, und der dritte (nichtdeckender Marker oder nDM) ein Emissionsspektrum aufweist, das die Spektren der beiden anderen Marker nicht überlappt (nicht deckendes Emissionsspektrum);
- Messen, mit einem beliebigen geeigneten Mittel, der Gesamtmenge an nichtdeckendem Marker (mnDM) in einem gewählten Detektionsband im Emissionsspektrum des genannten nichtdeckenden Markers;
- Messen, mit einem beliebigen geeigneten Mittel, der Gesamtmenge an deckenden Markern (mDM) in einem mit den Emissionsspektren der genannten deckenden Marker gemeinsamen Detektionsband;
- Ermitteln des Verhältnisses (V) zwischen der Gesamtmenge an nichtdeckenden Markern und der Gesamtmenge an deckenden Markern [R = (mnDM)/(mDM)];
- Erstellen, mit einem beliebigen Mittel, eines Diagramms, das das funktionelle Verhältnis (V) der Gesamtmenge an biologischen Elementen aufweist, die von dem deckenden Marker markiert wurden [V = f(mDM)].

2. Verfahren nach Anspruch 1, **dadurch gekennzeichnet, dass** es darüber hinaus eine letzte Quantifizierungsstufe, mit einem beliebigen Mittel, der Populationen der auf dem genannten Diagramm vorhandenen biologischen Elemente beinhaltet.

3. Verfahren nach Anspruch 1 oder 2, **dadurch gekennzeichnet, dass** die natürliche biologische Flüssigkeit Blut, Urin, abgelöstes Gewebe, Knochenmark, zerebrospinale Flüssigkeit, Tränenflüssigkeit, Synovialflüssigkeit, aus einem Aphäreseverfahren resultierendes Produkt ist.

4. Verfahren nach einem der Ansprüche 1 bis 3, **dadurch gekennzeichnet, dass** die in der Flüssigkeit eventuell vorhandenen biologischen Elemente eukaryotische Zellen oder prokaryotische Zellen oder ein Gemisch der beiden oder von Fragmenten der genannten Zellen sind.

5. Verfahren nach einem der Ansprüche 1 bis 4, **dadurch gekennzeichnet, dass** die Sonden, gleich oder unterschiedlich, Antikörper oder Nukleinsäuren (DNA oder RNA) oder spezielle Farbstoffe von Nukleinsäuren oder von enzymatischen Substraten oder auch spezielle Proteinmarker, Rezeptorliganden oder Marker, die für die ionische Umgebung empfindlich sind, oder ein beliebiges anderes Molekül sind, das für das biologische Zielelement charakteristisch ist.

6. Verfahren nach Anspruch 5, **dadurch gekennzeichnet, dass** die Antikörper monoklonale oder polyklonale Antikörper, natürlich oder rekombinant, menschlich oder tierisch, sind.

7. Verfahren nach einem der Ansprüche 1 bis 6, **dadurch gekennzeichnet, dass** die Marker, die dazu dienen, die Sonde erkennbar zu machen, Marker, interkalierend oder nicht, von Nukleinsäuren oder auch spezifische Proteinmarker oder von einem beliebigen anderen Molekül sind, das für das biologische Zielelement charakteristisch ist.

8. Verfahren nach einem der Ansprüche 1 bis 7, **dadurch gekennzeichnet, dass** die Marker fluoreszierend sind oder auch nicht.

9. Verfahren nach Anspruch 7 oder 8, **dadurch gekennzeichnet, dass** der Marker ausgewählt wird aus Alexa Fluor® 350, Alexa Fluor® 488, Alexa Fluor® 532, Alexa Fluor® 633, Alexa Fluor® 647, Alexa Fluor® 660, Alexa Fluor® 680, Allophycocyanin, Aminomethylcumarinessigsäure, Cy2®, Cy 5.1® Cy 5®, Cy 5.5®, Dichlorfluorescein (DCFH), Dihydrorhodamin (DHR), "enhanced GFP" (EGFP), Fluo-3, FluorX®, Fluorescein, 5-Maleimidfluorescein, Fluorescein-Isothiocyanat (FITC), PerCP, r-Phycoerythrin (PE), das Tandem r-Phycoerythrin-Cyanin 5 oder SpectralRed® oder CyChrome®, r-Phycoerythrin-Cyanin 5.5 (PE-CY 5.5®), r-Phycoerythrin-Cyanin 7 (PE-CY 7®), r-Phycoerythrin-Texas Red-x®, Red 613®, Rhodamin 110, Rhodamin 123, S65L, S65T, Tetramethylrhodamin-Isothiocyanat, Texas-Red-x®, TruRed®, Indo 1, Nanokristalle (Quantum Dots), Fura 2, Fura 3, Quin, DS Red, Interkalationsverbindungen wie Ethidiumbromid oder auch Thiazol Orange, Thiazol Blau, Thioflavin S, Thioflavin T, Thioflavin TCN®, Diethylchinolythocyaniniodid (DEQTC), TOTO-1®, TO-PRO-1® oder auch YOYO-1®, Hoechst® 33258, Hoechst® 33342, Hoechst® 34580, Diamidinophenylindol (DAPI), Propidiumiodid, Pyronine Y, 7-Aminoactinomycin D (7AAD), Acridin Orange, Auramin O, Calcein, New Methylene Blue, Olamin-O, Oxazin 750, Astrablau, SYTOX® Green, SYTO 11®, SYTO 12®, SYTO 13® SYTO 16®, SYTO 18®, SYTO 80®, SYTO 81®.

10. Anwendung des Verfahrens nach einem der vorherigen Ansprüche zum Unterscheiden von wenigstens zwei Populationen von biologischen Trägerelementen mit spezifischen Charakteristiken, die eventuell in einer Probe vorhanden sind.
